(19) Europäisches Patentamt
European Patent Office
Office européen des brevets

(11) Publication number: 0 230 085 A1

# (12) EUROPEAN PATENT APPLICATION

(21) Application number: 86202393.4

(51) Int. Cl.⁴: C07J 9/00

(22) Date of filing: 30.12.86

(30) Priority: 09.01.86 IT 1903886

(43) Date of publication of application:
29.07.87 Bulletin 87/31

(84) Designated Contracting States:
AT BE CH DE ES FR GB GR IT LI LU NL SE

(71) Applicant: BLASCHIM S.p.A.
Via Vittor Pisani, 28
I-20124 Milano(IT)

(72) Inventor: Magni, Ambrogio
Via Donizetti, 20
I-22058 Osnago Como(IT)
Inventor: Piccolo, Oreste
Via Cassa di Risparmi, 31
I-57100 Livorno(IT)
Inventor: Ascheri, Antonio
Via Don Sturzo, 4
I-20057 Vedano Al Lambro Mi(IT)

(74) Representative: Marchi, Massimo et al
c/o Marchi & Mittler s.r.l. Viale Lombardia 20
I-20131 Milano(IT)

## (54) Stereoselective reduction of the keto group at 7-position of a bile keto acid.

(57) The keto group at 7-position of a bile keto acid is stereoselectively reduced to beta-hydroxy group with hydrogen in the presence of nickel, of a base the quantity of which is of at least 0.3 mole to each mole of keto acid, and of an alcohol, having from 3 to 10 C atoms, selected from the group consisting of secondary alcohols, tertiary alcohols and beta-branched alcohols.

EP 0 230 085 A1

# "STEREOSELECTIVE REDUCTION OF THE KETO GROUP AT 7-POSITION OF A BILE KETO ACID"

This invention relates to a process for reducing stereoselectively the keto group at 7-position of a bile keto acid to beta hydroxy group with hydrogen in the presence of nickel, of a base the quantity of which is of at least 0.3 mole to each mole of keto acid, and of an alcohol, having from 3 to 10 C atoms, selected from the group consisting of secondary alcohols, tertiary alcohols and beta-branched alcohols.

The term "beta-branched alcohols" as used herein indicates a compound of the formula

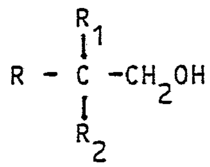

(III)

wherein R, $R_1$ and $R_2$, the same or different, are hydrogen, alkyl or phenyl, provided that at least two of the radicals R, $R_1$ and $R_2$ are different from hydrogen.

Preferably, the process of this invention relates to the reduction of a bile keto acid of the formula

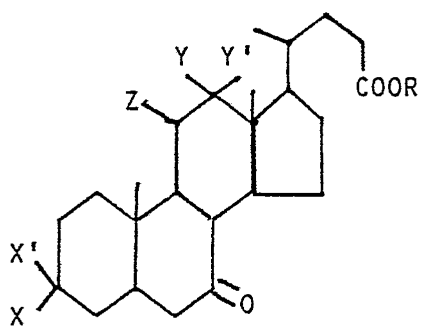

(I)

where R is H, an alkyl having from 1 to 10 C atoms, $Si(R")_3$, or a cation, wherein in turn R", the same or different, are alkyl having from 1 to 6 C atoms or phenyl;

X′ is OH, OR′, OCOH, OCOR′, $OSi(R")_3$, wherein in turn R′ is an alkyl having from 1 to 10 C atoms optionally substituted by phenyl or fluorine, or an aryl optionally substituted, and R" has the above mentioned meanings;

X is H or, together with X′, is an oxygen atom;

Y′ is H, OH, OR′, OCOH, OCOR′, $OSi(R")_3$, $OSO_2R'$ wherein R′ and R" have the above mentioned meanings;

Y is H or, together with Y′, is an oxygen atom, or, together with Z, is a covalent bond;

Z is H or, together with Y, has the above mentioned meaning;

to prepare a compound of the formula:

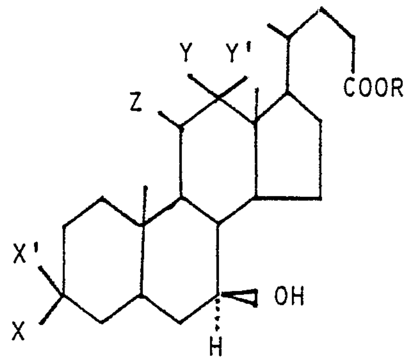

(II)

where R, X, X′, Y, Y′ and Z have the above mentioned meanings.

Specific examples of cations (R in the formula I) are sodium, potassium, calcium and magnesium.

Preferred meanings of R′ are methyl, ethyl, propyl, butyl, hexyl, trifluoromethyl, phenyl, p-methylphenyl, benzyl and naphthyl.

Preferred meanings of R" are methyl, ethyl, isopropyl, ter.butyl and phenyl.

It is known that the carbonyl group at 7-position of a bile keto acid can be converted into hydroxy group by means of various reducing systems.

In accordance with the reducing system which is used the resulting hydroxy group has alpha or beta configuration; an epimeric mixture is thus obtained when the reducing system is not stereoselective.

It is also known that the preparation of the alpha epimer in substantially pure form is not particularly difficult.

Suitable reducing agents for preparing the alpha epimer are, for example, sodium dithionite and sodium borohydride in basic medium (Tetr. Letters, 2487, 1983) and K-Selectride (Tetrahedron, 40 , 851, 1984).

On the contrary, a reducing system which affords the beta epimer in substantially pure form is not yet known.

They are only known some systems which afford epimeric mixtures wherein the amount of the beta epimer is prevailing.

Said reducing systems are consisting of a metal, such as sodium, potassium or zinc and an alcohol or liquid ammonia; the ratio of the beta to the alpha epimer ranges in accordance with the experimental conditions, the kind of solvent, the nature of the metal and the like (Angew. Chem. Int. Ed. Engl. 24, 499 (1985); Tetrahedron Letters 2487 (1983); Japanese Kokai Tokyo Koho 82 56,497 (C.A. 97, 127928s (1982); French Application 2,453,182 (C.A. 97, 163327d (1982); Spanish Patent 489,661 (C.A. 96 , 20375m (1982), Japanese Kokai 77 07,950 (C.A. 87, 1668274n); Acta Chem. Scand. 14, 17 (1960)).

The greatest stereoselectivity towards the beta epimer (about 95/5) is obtained when it is used an alkali metal, in particular potassium, and a tertiary alcohol.

However, this process requires special safety measures and can be performed only on a relatively small scale because of the known inflammability of the alkali metals which are used in great excess.

Therefore, there is still demand of a highly stereoselective, cheap and safe process for reducing the keto group at 7-position of a bile keto acid to beta-hydroxy group.

Now, it has been surprisingly found that this object can be achieved with hydrogen, in the presence of nickel, of a base the quantity of which is of at least 0.3 mole to each mole of keto acid, and of an alcohol, having from 3 to 10 C atoms selected from the group consisting of secondary alcohols, tertiary alcohols and beta-branched alcohols of the formula

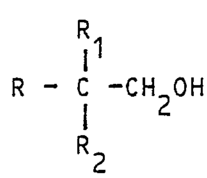

(III)

wherein R, $R_1$ and $R_2$, the same or different, are hydrogen, alkyl or phenyl, provided that at least two of the radicals R, $R_1$ and $R_2$ are different from hydrogen.

A further advantage of the process of this invention is that it is not required an especially pure nickel and it can be used a commercial nickel impure from other metals, such as the Raney nickel.

The results obtained by the process of this invention are even more unexpected when considering that:

a) in the presence of Raney nickel hydrogen reduces stereose lectively the keto group at 3-position and not that one at 7-position. In fact, the Japanese Patent Application 77/78864 (C.A. 87, 201897t (1977)) discloses a process for preparing ursodeoxycholic acid (UDCA) wherein in a first step the keto group at 3-position of a compound of formula I (wherein R = Z = Y = Y' = H; X + X' = O) is reduced with hydrogen and Raney nickel to give the corresponding compound wherein X is hydrogen and X' = alpha-OH without affecting the keto group at 7-position which is then reduced with potassium and ter.butanol to give a mixture consisting mostly of the beta-epimer;

b) as already seen before, when the Raney nickel is absent the hydrides afford the alpha-epimer; and

c) Raney nickel favours the epimerization of the steroid hydroxy groups, such as for example those at 3-position, possibly through formation of keto intermediates, and/or favours isomerization of the steroid rings A/B cis to A/B trans (J. Org. Chem. 33, 175 (1968); ibid. 33, 2814 (1968); ibid. 44, 4567 (1979); Tetrahedron Letters 2085 (1979)).

An essential feature of the process of this invention is the presence of a base the quantity of which is of at least 0.3 mole to each mole of keto acid.

The absence of the base spoils the system of its reducing capacity when hydrogen is used (Example 3) or causes mainly the formation of alpha epimer when a hydride is used (Example 2).

Quantities lower than 0.3 mole give unsatisfactory results both as to the yields and to the repeatability of the process.

The quantity of base is preferably comprised from 0.3 to 2 moles to each equivalent of keto group.

Examples of suitable bases are the alkali and alkaline earth metal alcoholates, phenates, carbonates, hydroxides and the like.

Examples of preferred bases are sodium and potassium ter. butylate, isopropylate, ter.amylate, methylate and phenate, potassium carbonate and hydroxide.

The amount of nickel with respect to the bile keto acid is preferably comprised from 0.1 to 2 (w/w).

The alcohols may be either alicyclic or cyclic. Examples of suitable alcohols are isopropyl, ter.butyl, sec.butyl, ter.amyl, isobutyl and neopentyl alcohol, 2-pentanol, 1-phenyl-ethanol, and cyclohexanol.

Hydrogen may also be generated "in situ" by suitable hydrogen donors. Examples of hydrogen donors are the secondary alcohols and the hydrides such as sodium and potassium borohydride, and tributyl tin hydride.

Preferred conditions are: as to temperature, 0°-150°C, still preferably 20°-85°C; as to pressure, 1-10 atm, still preferably from 1 to 4 atm; as to reaction time, 2-50 hrs.

According to this invention at least 90% of the keto group at 7-position is reduced and the obtained product contains up to 98% of beta-epimer.

When the bile keto acid contains other keto groups, such as for example at 3 and/or 12-position, they also may be reduced with the process of this invention. In such a case, the amount of the reactants will be adjusted in accordance with the expected result and the base may amount to 3 mole for each mole of keto acid.

When carrying out the process of this invention, the ester groups which may be present in compound (I) may undergo hydrolization and the group $OSO_2R'$ may undergo elimination with formation of a double bond.

The advantages offered by this invention will become evident to the artisan through the present disclosure and the following examples which are intended to illustrate this invention without limiting it.

EXAMPLE 1

Potassium 3alpha-hydroxy-7-oxo-5beta-cholanate (9.05 g; 21.1 mmol), isopropyl alcohol (450 ml) and potassium ter.butilate (2.74 g; 24.4 mmol) were added to Raney nickel (9.5 g) in nitrogen atmosphere. Hydrogen was added and the reaction mixture was maintained at 40°C and at atmospheric pressure while monitoring the reaction course by HPLC analysis.

When the reaction was complete, stirring and heating were stopped. The alcoholic phase was separated and the solvent removed by evaporation.

The residue was treated with water (200 ml) and the thus obtained solution was made acid with 1N hydrochloric acid. The precipitate was collected by filtration, washed with water and dried.

A mixture (7.90 g; Yield, 95%) of ursodeoxycholic acid (UDCA; 7.63 g; 97%) and of chenodeoxycholic acid (CDCA; 0,27 g; 3%) was thus obtained.

Analogous results were obtained by working as indicated before but replacing:
-isopropyl alcohol with ter.butyl alcohol and heating at 80° instead of at 40°C;
-isopropyl alcohol with pentan-2-ol and heating at 60°C instead of at 40°C;
-isopropyl alcohol with sec.butyl or isobutyl alcohol;
-isopropyl alcohol with neopentyl alcohol and heating at 55°C instead of at 40°C;
-potassium ter.butylate with sodium hydroxyde, potassium car bonate or phenate;
-Raney nickel with commercially pure nickel without adding hydrogen and using isopropyl alcohol as hydrogen donor at 81°C;
-hydrogen with potassium borohydride (3 g; 55.6 mmol).

EXAMPLE 2

Potassium 3alpha-7-oxo-5beta-cholanate (2 g; 4.67 mmol) was added to a suspension of Raney nickel - (2 g) in isopropyl alcohol (80 ml). Potassium borohydride (0.63 g; 11.68 mmol) was then added.

The mixture was kept under stirring at 40°C until the reduction was over (HPLC analysis).

Finally the reaction mixture was treated as indicated in Example 1 above.

A mixture containing 65% of CDCA, 10% of UDCA and 25% of the starting product was thus obtained.

EXAMPLE 3

No reduction product was obtained by working as indicated in Example 1 above but leaving out potassium ter.butylate.

EXAMPLE 4

Potassium 3,7-dioxo-5beta-cholanate (2 g; 4.7 mmol), isopropyl alcohol (80 ml) and potassium ter.butylate (1.15 g; 10.3 mmol) were added to Raney nickel (2 g) in nitrogen atmosphere. Hydrogen was added and the reaction mixture was maintained at 40°C and at atmospheric pressure while monitoring the reaction course by HPLC analysis.

When the reduction was over, stirring and heating were stopped. The alcoholic phase was separated and the solvent was removed by evaporation.

The residue was treated with water (40 ml) and the thus obtained solution was made acid with 1N hydrochloric acid. The precipitate was collected by filtration, washed with water and dried.

A mixture (1.75 g) of UDCA (98%) and CDCA (2%) was thus obtained. The conversion was 96%.

EXAMPLE 5

The process of the Example 4 was repeated replacing potassium 3,7-dioxo-5beta-cholanate with trimethylsilyl ester of 3alpha-trimethylsilyloxi-7-oxo-5beta-cholanic acid (1.8 g; 3.3 mmol) and using 0.92 g - (8.2 mmol) of potassium ter.butylate.

A mixture (1.21 g) was thus obtained containing 3% of 3alpha-hydroxy-7-oxo-5beta-cholanic and 97% of UDCA/CDCA (96/4).

EXAMPLE 6

Potassium 3alpha-hydroxy-7-oxo-5beta-cholanate (3.6 g; 8.4 mmol), isopropyl alcohol (120 ml) and potassium isopropylate (0.25 g; 2.5 mmole) were added to commercially pure nickel (2.8 g) in nitrogen atmosphere.

The reaction mixture was refluxed for 2 hours and then worked up as disclosed in Example 4 above.

The conversion was 60%; UDCA/CDCA = 94/6.

The process was repeated with:
-0.42 g (4.1 mmol) of potassium isopropylate; conversion, 80%; UDCA/CDCA = 95/5;
-0.84 (8.2 mmol) of potassium isopropylate and refluxing the reaction mixture for 3 hrs; conversion, 93%; UDCA/CDCA = 95/5.

## Claims

1. A process for reducing stereoselectively the keto group at 7-position of a bile keto acid to beta hydroxy group, characterized in that the keto acid is reduced with hydrogen in the presence of nickel, of a base the quantity of which is of at least 0.3 mole to each mole of keto acid, and of an alcohol, having from 3 to 10 C atoms, selected from the group consisting of secondary alcohols, tertiary alcohols and beta-branched alcohols of the formula

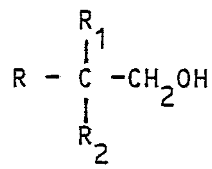

(III)

wherein R, $R_1$ and $R_2$, the same or different, are hydrogen, alkyl, or phenyl, provided that at least two of the radicalsR, $R_1$ and $R_2$ are different from hydrogen.

2. A process according to claim 1 above, characterized in that a bile keto acid of the formula:

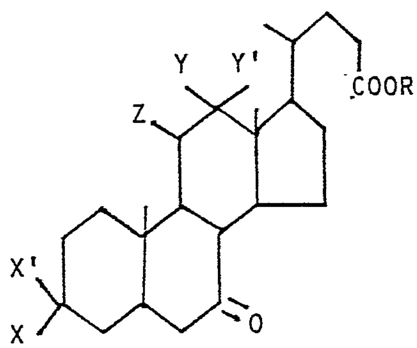

(I)

where R is H, an alkyl having 1-10 C atoms, $Si(R'')_3$, or a cation, wherein in turn R", the same or different, are alkyl having from 1 to 6 C atoms or phenyl;
X' is OH, OR', OCOH, OCOR', $OSi(R'')_3$ wherein in turn R' is an alkyl having 1-10 C atoms, optionally substituted by phenyl or fluorine, or an aryl optionally substituted, and R" has the above mentioned meanings;
X is H or , together with X', is an oxygen atom;
Y' is H, OH, OR', OCOH, OCOR', $OSi(R'')_3$, $OSO_2R'$ wherein R' and R" have the above mentioned meanings;
Y is H or, together with Y', is an oxygen atom or, together with Z, is a covalent bond;
Z is H or, together with Y, has the above mentioned meaning.
is reduced to prepare a compound of the formula:

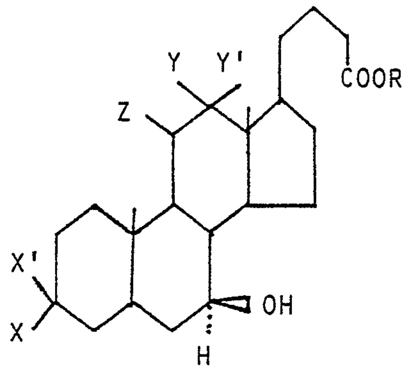

(II)

wherein R, X, X′, Y, Y′ and Z have the above mentioned meanings.

3. A process according to claim 2 above, characterized in that 3alpha-hydroxy-7-oxo-5beta-cholanic acid and 3,7-dioxo-5beta-cholanic acid are reduced to ursodeoxycholic acid.

4. A process according to one or more claims from 1 to 3 above, characterized in that hydrogen is generated "in situ" by a hydrogen donor.

5. A process according to claim 4 above, characterized in that the hydrogen donor is selected from the group comprising sodium borohydride, potassium borohydride, tributyl tin hydride, and secondary alcohols.

6. A process according to one or more of claims from 1 to 5 above, characterized in that a commercially pure nickel or Raney nickel is used.

7. A process according to one or more of claims from 1 to 6 above, characterized in that the base is an alcoholate, a phenate, a carbonate or a hydroxide of an alkali or alkaline earth metal.

8. A process according to claim 7 above, characterized in that base is a sodium or potassium alcoholate, phenate, hydroxide or carbonate.

9. A process according to one or more of claims from 1 to 8 above, characterized in that the reaction is carried out at a temperature comprised from 0°C to 150°C.

10. Process according to claim 9 above, characterized in that the reaction is carried out at a temperature comprised from 20° to 85°C.

11. Process according to one or more of claims from 1 to 10, characterized in that the reaction is carried out at a pressure comprised from 1 to 10 atom.

12. Process according to claim 11 above, characterized in that the reaction is carried out at from 1 to 4 atm.

European Patent Office

# EUROPEAN SEARCH REPORT

Application number

EP 86 20 2393

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | PATENT ABSTRACTS OF JAPAN, vol. 6, no. 131 (C-114)[1009], 17th July 1982; & JP-A-57 56 497 (TOUKIYOU TANABE SEIYAKU K.K.) 05-04-1982<br>* Abstract *<br>--- | 1,6 | C 07 J 9/00 |
| Y | ANGEWANDTE CHEMIE INTERNATIONAL EDITION IN ENGLISH, vol. 24, no. 6, June 1985, pages 499-500, VCH Verlagsgesellschaft mbH, Weinheim, DE; C. GIORDANO et al.: "Reduction of ketones by alkali metals in tertiary alcohols: Unexpected cation effect on the stereochemistry"<br>* Whole article *<br>--- | 1 | |
| A | GB-A-2 036 749 (ROUSSEL-UCLAF)<br>* Whole patent *<br>--- | 1 | TECHNICAL FIELDS SEARCHED (Int. Cl.4)<br>C 07 J 9/00 |
| D,A | CHEMICAL ABSTRACTS, vol. 87, no. 21, 21st November 1977, page 603, abstract no. 168274n, Columbus, Ohio, US; & JP-A-77 07 950 (TOKYO TANABE CO. LTD) 21-01-1977<br>* Abstract *<br>----- | 1 | |

The present search report has been drawn up for all claims

| Place of search | Date of completion of the search | Examiner |
|---|---|---|
| THE HAGUE | 10-04-1987 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document